(19) **Europäisches Patentamt**
**European Patent Office**
**Office européen des brevets**

(11) **EP 2 589 291 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**21.08.2019 Patentblatt 2019/34**

(45) Hinweis auf die Patenterteilung:
**07.12.2016 Patentblatt 2016/49**

(21) Anmeldenummer: **12188081.9**

(22) Anmeldetag: **11.10.2012**

(51) Int Cl.:
*A01N 31/14* (2006.01)      *A01N 31/02* (2006.01)

(54) **Synergistisch wirksame ternäre antimikrobielle Mischungen**

Synergistically active ternary anti-microbial mixtures

Mélanges antimicrobiens ternaires à efficacité synergétique

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.11.2011 DE 102011085798**

(43) Veröffentlichungstag der Anmeldung:
**08.05.2013 Patentblatt 2013/19**

(73) Patentinhaber: **Symrise AG**
**37603 Holzminden (DE)**

(72) Erfinder:
• **Pillai, Ravikumar**
**Emerson, NJ New Jersey 07630 (US)**
• **Köhler, Antje**
**37603 Holzminden (DE)**
• **Schmaus, Gerhard**
**37671 Höxter-Bosseborn (DE)**

(74) Vertreter: **Fabry, Bernd**
**IP2 Patentanwalts GmbH**
**Schlossstrasse 523-525**
**41238 Mönchengladbach (DE)**

(56) Entgegenhaltungen:
**WO-A1-03/069994      WO-A1-2005/102276**
**DE-U1- 20 221 386**

Bemerkungen:
Die Akte enthält technische Angaben, die nach dem Eingang der Anmeldung eingereicht wurden und die nicht in dieser Patentschrift enthalten sind.

EP 2 589 291 B2

**Beschreibung**

Die vorliegende Erfindung betrifft das Gebiet der antimikrobiellen Wirkstoffe und insbesondere bestimmte Mischungen, Zubereitungen und Nahrungsmittel umfassend 2-Phenoxyethanol, sowie wenigstens zwei verschiedene Alkandiole, ausgewählt aus der Gruppe bestehend aus 1,2-Decandiol, 1,2-Octandiol, 1,2-Hexandiol und 1,2-Pentandiol. Die Erfindung betrifft ferner die Verwendung von 2-Phenoxyethanol zur synergistischen Verstärkung der antimikrobiellen Wirksamkeit von Mischungen aus wenigstens zwei der vorgenannten Alkandiolen, ein Verfahren zur Konservierung oder antimikrobiellen Behandlung eines verderblichen Produktes, ein Verfahren zur kosmetischen Behandlung bestimmter Mikroorganismen sowie die Verwendung einer erfindungsgemäßen Mischung zur therape u-tischen Behandlung bestimmter Mikroorganismen.

In der kosmetischen und pharmazeutischen sowie in der Lebensmittelindustrie besteht ein beständiger Bedarf an Mitteln mit antimikrobiellen Eigenschaften, insbesondere zur Konservierung ansonsten verderblicher Produkte (wie z.B. Kosmetika, pharmazeutische Produkte oder Lebensmittel), aber auch zur unmittelbaren kosmetischen oder therapeutischen Behandlung von Mikroorganismen, welche einen nachteiligen Einfluss auf den menschlichen oder tierischen Körper besitzen können. Exemplarisch hingewiesen sei auf Mikroorganismen, die Körpergeruch, Akne, Mykosen oder dergleichen verursachen können.

Zwar werden in den angesprochenen technischen Bereichen bereits eine Vielzahl antimikrobieller Wirkstoffe eingesetzt, doch wird weiterhin nach Alternativen gesucht, um gezielte Spezialbehandlungen durchführen und/oder Nebenwirkungen reduzieren zu können. Bei der Suche nach alternativen antimikrobiell und insbesondere konservierend wirkenden Mitteln ist dabei allerdings zu beachten, dass die im kosmetischen, pharmazeutischen und/oder Nahrungsmittelbereich verwendeten Substanzen

- toxikologisch unbedenklich,

- gut hautverträglich,

- stabil (insbesondere in den üblichen kosmetischen und/oder pharmazeutischen Formulierungen),

- weitgehend und vorzugsweise vollständig geruchlos und/oder

preiswert herstellbar (d.h. unter Einsatz von Standardverfahren und/oder ausgehend von Standardprekursoren) sein müssen. Ferner ist es wünschenswert, in den entsprechenden Mitteln möglichst wenig der antimikrobiellen Wirkstoffe einsetzen zu müssen, um einen bestimmten antimikrobiellen Effekt zu erzielen.

Die Suche nach geeigneten (Wirk-)Substanzen, die eine oder mehrere der genannten Eigenschaften in ausreichendem Maße besitzen, ist dem Fachmann dadurch erschwert, dass keine klare Abhängigkeit zwischen der chemischen Struktur einer Substanz einerseits und ihrer biologischen Aktivität gegenüber bestimmten Mikroorganismen (Keimen) sowie ihrer Stabilität andererseits besteht. Des Weiteren gibt es keinen vorhersehbaren Zusammenhang zwischen der antimikrobiellen Wirkung, der toxikologischen Unbedenklichkeit, der Hautverträglichkeit und der Stabilität einer Substanz.

Aufgabe der vorliegenden Erfindung war es daher, Mittel anzugeben, die mehrere oder alle oben genannten Vorgaben erfüllen und/oder eine wünschenswerte Kombination der oben genannten Eigenschaften besitzen.

Diese Aufgabe wird gelöst durch eine Mischung umfassend oder bestehend aus

(a) 2-Phenoxyethanol und

(b1) 1,2-Decandiol und (b2) 1,2-Pentandiol.

Die Erfindung beruht auf der überraschenden Erkenntnis, dass die erfindungsgemäßen ternären Mischungen einen synergistisch verstärkten antimikrobiellen Effekt zumindest gegenüber ausgewählten Keimen zeigen können, insbesondere gegenüber *Aspergillus niger,* einem nur sehr schwer zu bekämpfenden Schimmelpilz.

Insbesondere hat sich gezeigt, dass die erfindungsgemäße Mischung hervorragend als antimikrobielle Wirkstoffmischung, insbesondere zur Konservierung ansonsten verderblicher Artikel (siehe oben) verwendet werden kann.

Obwohl sich die Fachwelt bereits umfangreich mit den antimikrobiellen Eigenschaften von 1,2-Alkandiolen und 2-Phenoxyethanol befasst hat, gab es bislang keinen Hinweis, dass die erfindungsgemäßen ternären Mischungen solcher Verbindungen eine wenigstens im Einzelfall deutlich verbesserte antimikrobielle Wirkung (zumindest gegenüber ausgewählten Keimen) besitzen.

Die antimikrobielle Wirkung von 2-Phenoxyethanol, einem weit verbreitetem Konservierungsmittel ist bekannt und z. B. im "Handbuch der Konservierungsmittel" (Hrsg.: Deutsche Gesellschaft für wissenschaftliche und angewandte Kosmetik, Verlag für chemische Industrie, H.Ziolkowsky GmbH, D-86150 Augsburg, 1995) beschrieben. Untersuchungen zu einer synergistisch verstärkten Wirksamkeit ternärer Kombination mit 1,2-Decandiol und 1,2-Hexandiol insbesondere gegenüber *Aspergillus niger* sind jedoch bis dato in keiner Schrift offenbart.

Die antimikrobielle Wirkung von Polyolen, insbesondere von aliphatischen 1,2-Diolen und Kombinationen von 1,2-Diolen mit weiteren antimikrobiell wirksamen Stoffen ist in diversen Dokumenten beschrieben. Beispielhaft erwähnt seien hier JP5191327, JP11322591, EP1206933, WO 03/069994. Untersuchungen zu einer synergistisch verstärkten Wirksamkeit gegenüber *Aspergillus niger* bei Kombination mit 2-Phenoxyethanol sind jedoch in keiner dieser Schriften offenbart.

WO 2005/102276 A1 offenbart eine antimikrobielle Zusammensetzung, die aus einer Mischung von 40 bis 60% 1,2-Alkandiolen und 40 bis 60% Phenoxyethanol besteht, wobei als 1,2-Pentandiol, 1,2-Hexandiol und 1,2-Octandiol als 1,2-Diole eingesetzt werden.

WO 03/069994 A1 und DE 20221386 U1 gehören zur selben Patentfamilie und betreffen eine antimikrobielle Zusammensetzung aus einer Mischung aus 1,2- Alkandiolen. Als bevorzugte1,2-Alkandiol- Mischungen werden genannt:

- 1,2-Hexandiol und 1,2- Octandiol,

- 1,2-Hexandiol und 1,2- Decandiol,

- 1,2- Pentandiol, 1,2-Hexandiol und 1,2- Octandiol,

- 1,2-Hexandiol und 1,2- Octandiol und 1,2- Decandiol, oder

- 1,2- Pentandiol, 1,2-Hexandiol und 1,2- Decandiol.

In den Beispielen werden C6/C8 Gemische offenbart, wobei in Tabelle 8 und 9 zu dem C6/C8- Gemisch ein Konservierungsmittel Euxyl K400, dass ein Gemisch aus (20%) 1,2-Dibrom-2,4-dicyanbutan und (80%) 2-Phenoxyethanol besteht.

Polyole und insbesondere 1,2-Alkandiole sind zumeist gegenüber Schimmelpilzen wie *Aspergillus niger* nur mangelhaft wirksam. Hinsichtlich einzelner Polyole oder Mischungen von Polyolen ist also eine Lücke in der Wirksamkeit bei Schimmelpilzen (z.B. dem "Problemkeim" *Aspergillus niger*) zu verzeichnen. Zur kompletten Inhibierung von Schimmelpilzen sind daher bislang hohe Einsatzkonzentrationen einzelner Polyole oder von Mischungen aus Polyolen notwendig.

Es war daher besonders überraschend, dass die erfindungsgemäße Mischung eine stark synergistische Wirksamkeit zeigt. Sie ist zum Beispiel bei der Behandlung von *Aspergillus niger* im Hinblick auf die Keimzahlreduktion bereits nach 48 Stunden deutlich überlegen, wenn sie bei gleicher Einsatzkonzentration in den vor mikrobiellen Befall zu schützenden Endprodukten enthalten ist. Insbesondere wurde bei gegebenen Konzentrationen nur durch die besagte erfindungsgemäße ternäre Mischung eine Keimzahlreduktion um annähernd 2 log Stufen bereits nach 48h erreicht.

Die erfindungsgemäße Mischung ist auf Grund der besonders signifikanten Wirkungsverstärkung ihrer Bestandteile insbesondere zur Bekämpfung von *Aspergillus niger* selbst bei niedriger Dosierung der erfindungsgemäßen Mischung geeignet.

Erfindungsgemäß bevorzugt in diesem Sinne ist die Verwendung einer erfindungsgemäßen Mischung, umfassend oder bestehend aus

(a) 2-Phenoxyethanol,

(b1) 1,2-Decandiol und

(b2) 1,2-Pentandiol,

zur Konservierung und antimikrobiellen Behandlung verderblicher Produkte gegen *Aspergillus niger* wobei das Gewichtsverhältnis der Komponente (a) zur Komponente (b1+b2) 0,3-1,2 zu 0,06-0,7 beträgt.

Dabei ist besonders bevorzugt eine erfindungsgemäße Mischung, bei der bei das Gewichtsverhältnis der Komponente (a) zur Komponente (b1 + b2) 0,3 - 1,2 zu 0,06 - 0,7 beträgt, und insbesondere bei der die Gewichtsverhältnisse der Komponenten (a) zu (b1) zu (b2) 0,3 -1,2 zu 0,05 - 0,4 zu 0,01 - 0,3 betragen.

Besonders bevorzugt ist eine erfindungsgemäße Mischung, bei der die Gewichtsverhältnisse der Komponenten (a) zu (b1 + b2) und/oder (a) zu (b1) zu (b2) so eingestellt sind, dass die antimikrobielle Wirkung synergistisch verstärkt ist.

Durch die geeignete Auswahl der Konzentrationen der Komponenten (a), (b1) und (b2) sowie die Auswahl der Mischungsverhältnisse der Komponenten zueinander lässt sich somit ein synergistischer Effekt hinsichtlich der antimikrobiellen Wirkung erzielen. Dies war - wie bereits oben angedeutet - überraschend. Dabei hat der synergistische Effekt den Vorteil, dass bei gleicher Wirksamkeit weniger antimikrobieller Wirkstoff eingesetzt werden muss bzw. bei gleicher Menge an Wirkstoff ein verbesserter antimikrobieller Effekt erzielt werden kann.

Eine antimikrobielle Wirksamkeit im Sinne dieses Textes liegt im Zweifelsfalle vor, wenn eine ausreichende Konservierung gemäß dem Europäischen Arzneibuch (ISBN 3-7692-2768-9; Nachtrag 2001 zur 3. Ausgabe, Seite 421-422, Kapitel 5.1.3) festgestellt wird. Der dabei bevorzugt zu verwendende Keim ist bevorzugt *Aspergillus niger* ATCC16404. Hinsichtlich weiterer Informationen zur Durchführung der Prüfung auf antimikrobielle Wirksamkeit wird insbesondere auch auf das weiter unten stehende Beispiel 1 verwiesen.

Eine synergistische Verstärkung (der antimikrobiellen Wirkung) liegt dann vor, wenn der Synergieindex (SI-Wert) der zu prüfenden Mischung nach Kull (Literatur: F.C.Kull et al, Applied Microbiology Vol.9, p. 538-541 (1961); D.C.Steinberg, Cosmetics & Toiletries Vol.115(11), 59-62 (2000)) einen Wert < 1 ergibt. Hinsichtlich weiterer Informationen zur Berechnung des Synergieindexes wird ebenfalls auf das Beispiel 1, weiter unten verwiesen. Auch hier ist wieder der bevorzugt einzusetzende Keim für die Bestimmung des Synergieindexes der oben bezeichnete *Aspergillus niger*-Stamm.

Die offenbarte antimikrobielle Mischung ist zur Konservierung und antimikrobiellen Behandlung verderblicher Produkte wie z.B. Kosmetikprodukten, pharmazeutischen Produkten oder Lebensmitteln geeignet. Dabei wird das verderbliche Produkt mit einer antimikrobiell wirksamen, vorzugsweise einer gegen *Aspergillus niger* wirksamen Menge der offenbarten Mischung kontaktiert.

Die erfindungsgemäße Mischung entfaltet ihre synergistisch verstärkte antimikrobielle Wirkung gegenüber einer Vielzahl von gram-positiven Bakterien, gram-negativen Bakterien, Schimmelpilzen und Hefen. Eine besonders gute Wirkung besteht gegenüber gram-negativen Bakterien wie *Escherichia coli* und *Pseudomonas aeruginosa,* gegenüber Hefen wie *Candida albicans* und eben - wie bereits erwähnt - gegenüber Pilzen wie *Aspergillus niger.* Als besonders vorteilhaft ist hierbei die sehr gute Wirksamkeit der erfindungsgemäßen Mischung gegenüber *Aspergillus niger,* einem nur sehr schwer zu bekämpfenden Schimmelpilz, zu betrachten.

Teil der Erfindung ist auch die Verwendung einer erfindungsgemäßen Mischung zur therapeutischen Behandlung von

Körpergeruch verursachenden Mikroorganismen,

Akne verursachenden Mikroorganismen und/oder

Mykosen verursachenden Mikroorganismen,

wobei die Mikroorganismen *Aspergillus niger* sind.

Bevorzugte Ausgestaltungen der erfindungsgemäßen Verfahren entsprechen den vorstehend erläuterten bevorzugten Ausgestaltungen der erfindungsgemäßen Verwendung.

Die menschliche Haut wird von einer Vielzahl verschiedener Mikroorganismen besiedelt, zu denen die vorstehend bereits genannten Mikroorganismen ebenso wie andere gehören. Die meisten dieser Mikroorganismen sind nicht pathogen und für den physiologischen Zustand der Haut und für deren Geruch irrelevant. Andere hingegen können den gesunden Zustand der Haut maßgeblich beeinflussen.

Wie eigene Untersuchungen jetzt zeigten, sind die erfindungsgemäßen synergistisch wirksamen Mischungen nicht nur gegenüber den vorstehend bereits bezeichneten Keimen, sondern auch gegenüber *Staphylococcus epidermidis, Corynebacterium xerosis, Brevibacterium epidermidis, Propionibacterium acnes* sowie gegenüber *Trichophyton*- und *Epidermophyton*-Arten gut wirksam, so dass sie auch als Mittel zur Behandlung (Bekämpfung) von Achsel- und Fußgeruch bzw. Körpergeruch im allgemeinen, als Mittel zur Bekämpfung von Akne, als Antischuppenmittel und zur Behandlung von Mykosen (insbesondere Dermatomykosen) eingesetzt werden können.

Unter "Behandlung" wird dabei im Rahmen des vorliegenden Textes jede Form der therapeutischen oder nichttherapeutischen Einflussnahme auf die betreffenden Mikroorganismen verstanden, bei der die Vermehrung dieser Mikroorganismen gehemmt und/oder die Mikroorganismen getötet werden.

Bevorzugt ist dabei eine erfindungsgemäße kosmetische oder pharmazeutische Zubereitung oder ein erfindungsgemäßes Nahrungsmittel, wobei die Gesamtmenge an den Komponenten (a) und (b1 + b2) und/oder Gesamtmenge an den Komponenten (a), (b1) und (b2) im Bereich von 0,01 bis 10 Gew.-% liegt, bezogen auf die Gesamtmasse der Zubereitung oder des Nahrungsmittels.

Bestandteil der Erfindung ist dabei eine erfindungsgemäße kosmetische oder pharmazeutische Zubereitung oder ein erfindungsgemäßes Nahrungsmittel enthaltend eine Mischung bestehend aus

0,30 bis 1,20 Gew.-% 2-Phenoxyethanol

0,05 bis 0,40 Gew.-% 1,2-Decandiol und

0,01 bis 0,20 Gew.-% 1,2-Pentandiol,

bezogen auf die Gesamtmasse der Zubereitung oder des Nahrungsmittels.

Darüber hinaus ist weiter bevorzugt eine erfindungsgemäße kosmetische oder pharmazeutische Zubereitung oder ein erfindungsgemäßes Nahrungsmittel, umfassend als Komponente (a) 2-Phenoxyethanol, als Komponente (b1) 1,2-Decandiol und als Komponente (b2) 1,2-Pentandiol mit einem Mengenanteil von (a) 70 Gew.-% 2-Phenoxyethanol, (b1) 0,20 Gew.-% 1,2-Decandiol und 0,1 Gew.-% 1,2-Pentandiol, bezogen auf die Gesamtmasse der Zubereitung oder des Nahrungsmittels.

Dabei sind bei den gerade beschriebenen erfindungsgemäßen Zubereitungen oder erfindungsgemäßen Nahrungsmitteln mit zunehmender Bevorzugung die besonderen antimikrobiellen Effekte, insbesondere auch die synergistische Verstärkung dieser Effekte jeweils weiter verbessert.

Dementsprechend ist darüber hinaus ganz besonders bevorzugt eine erfindungsgemäße kosmetische oder pharmazeutische Zubereitung oder ein erfindungsgemäßes Nahrungsmittel, wobei die Konzentration von jeder der Komponenten (a), (b1) und (b2) für sich unterhalb der antimikrobiell wirksamen Konzentration liegt, aber die Gesamtkonzentration der Komponenten (a), (b1) und (b2) antimikrobiell wirksam ist. In diesem Zusammenhang ist es besonders bevorzugt, dass die Mengenanteile der Komponente (a) im Bereich von 0,5 bis 1 Gew.-%, die der Komponente (b1) im Bereich von 0,1 bis 0,3 Gew.-% und die der Komponente (b2) im Bereich von 0,05 bis 0,2 Gew.-% liegen.

Die synergistisch wirksamen Mischungen können hierbei (a) prophylaktisch oder (b) im Bedarfsfall zum Einsatz kommen.

Die Konzentration der z.B. täglich zu applizierenden Wirkstoffmenge ist unterschiedlich und hängt vom physiologischen Zustand des Probanden sowie individualspezifischen Parametern wie Alter oder Körpergewicht ab. Die erfindungsgemäßen synergistisch wirksamen Mischungen können sowohl allein als auch in Kombination mit weiteren antimikrobiell wirksamen Substanzen zum Einsatz gelangen.

Es sei darauf hingewiesen, dass die erfindungsgemäß einzusetzenden 1,2-Alkandiole im Rahmen des vorliegenden Textes sowohl als entsprechendes 2S-konfiguriertes Enantiomer als auch als 2R-konfiguriertes Enantiomer sowie in Form beliebiger Mischungen dieser 2S- und 2R-konfigurierten Enantionmere vorliegen können. Aus kommerziellen Gründen ist es zwar besonders vorteilhaft, Gemische von Racematen der jeweiligen erfindungsgemäß einzusetzenden 1,2-Alkandiole zur Bekämpfung von Mikroorganismen einzusetzen, da diese synthetisch besonders leicht zugänglich sind, die reinen Enantiomere oder nicht-racemische Mischungen dieser Enantiomere sind aber ebenfalls für die erfindungsgemäßen Zwecke geeignet.

Weitere erfindungsgemäße Verwendungen/Verfahren und Mischungen/ Zusammensetzungen lassen sich den nachfolgenden Ausführungen und den beigefügten Patentansprüchen entnehmen.

Zubereitungen, die eine erfindungsgemäße Mischung enthalten, werden, insbesondere soweit sie gegen Körpergeruch verursachende Keime eingesetzt werden, in der Regel topisch in Form von Lösungen, Cremes, Lotionen, Gelen, Sprays oder dergleichen appliziert. Für andere Zwecke ist in manchen Fällen eine orale (Tabletten, Kapseln, Pulver, Tropfen), intravenöse, intraokulare, intraperitoneale oder intramuskuläre Applikation oder eine Applikation in Form eines imprägnierten Verbands sinnvoll.

Die erfindungsgemäßen Mischungen lassen sich ohne Schwierigkeiten in gängige kosmetische oder dermatologische Formulierungen (Zubereitung) wie u.a. Pumpsprays, Aerosolsprays, Cremes, Salben, Tinkturen, Lotionen, Nagelpflegeprodukte (z.B. Nagellacke, Nagellackentferner, Nagelbalsame) und dergleichen einarbeiten. Hierbei ist es auch möglich und in manchen Fällen vorteilhaft, die erfindungsgemäßen (synergistischen) Mischungen mit weiteren Wirkstoffen zu kombinieren, beispielsweise mit anderen antimikrobiell, antimykotisch bzw. antiviral wirksamen Stoffen. Die die erfindungsgemäßen (synergistischen) Mischungen enthaltenden kosmetischen und/oder dermatologischen/keratologischen Formulierungen können hierbei ansonsten wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Werden die erfindungsgemäßen Mischungen als Wirkstoffe zur Konservierung organischen Materials eingesetzt, so können vorteilhaft zusätzlich ein weiteres oder mehrere weitere Konservierungsmittel eingesetzt werden. Vorzugsweise gewählt werden hierbei Konservierungsmittel wie Benzoesäure, ihre Ester und Salze, Propionsäure und ihre Salze, Salicylsäure und ihre Salze, 2,4-Hexadiensäure (Sorbinsäure) und ihre Salze, Formaldehyd und Paraformaldehyd, 2-Hydroxybiphenylether und seine Salze, 2-Zinksulfidopyridin-N-oxid, anorganische Sulfite und Bisulfite, Natriumjodat, Chlorbutanolum, 4-Ethylquecksilber-(II)5-Amino-1,3-bis(2-Hydroxybenzoesäure, ihre Salze und Ester, Dehydratcetsäure, Ameisensäure, 1,6-Bis(4-amidino-2-bromphenoxy)-n-hexan und seine Salze, das Natriumsalz der Ethylquecksilber-(II)-thiosalicylsäure, Phenylquecksilber und seine Salze, 10-Undecylensäure und ihre Salze, 5-Amino-1,3-bis(2-ethylhexyl)-5-methyl-hexahydropyrimidin, 5-Brom-5-nitro-1,3-dioxan, 2-Brom-2-nitro-1,3-propandiol, 2,4-Dichlorbenzylalkohol, N-(4-Chlorphenyl)-N'-(3,4-dichlorphenyl)-harnstoff, 4-Chlor-m-kresol, 2,4,4'-Trichlor-2'-hydroxy-diphenylether, 4-Chlor-3,5-dimethylphenol, 1,1'-Methylen-bis(3-(1-hydroxymethyl-2,4-dioximidazolidin-5-yl)harnstoff), Poly-(hexamethylendiguanid)-hydrochlorid, 2-Phenoxyethanol, Hexamethylentetramin, 1-(3-Chloroallyl)-3,5,7-triaza-1-azonia-adamantanchlorid, 1(4-Chlorphenoxy)1(1H-imidazol-1-yl)-3,3-dimethyl-2-butanon, 1,3-Bis-(hydroxy-methyl)-5,5-dimethyl-2,4-imidazolidindion, Benzylalkohol, Octopirox, 1,2-Dibrom-2,4-dicyanobutan, 2,2'-Methylen-bis(6-brom-4-chlorphenol), Bromchlorophen, Mischung von 5-Chlor-2-methyl-3(2H)-isothiazolinon und 2-Methyl-3(2H)isothiazlinon mit Magnesiumchlorid und Magnesiumnitrat, 2-Benzyl-4-chlorphenol, 2-Chloracetamid, Chlorhexidin, Chlorhexidinacetat, Chlorhexidingluconat, Chlorhexidinhydrochlorid, 1-Phenoxy-propan-2-ol, N-Alkyl($C_{12}$-$C_{22}$)trimethylammoniumbromid und -chlorid, 4,4-Dimethyl-1,3-oxazo-lidin, N-Hydroxymethyl-N-(1,3-di(hydroxymethyl)-2,5-dioxoimidazolidin-4-yl)-N'-hydroxymethylharnstoff, 1,6-Bis(4-amidinophenoxy)-n-hexan und seine Salze, Glutaraldehyd, 5-Ethyl-1-aza-3,7-dioxabicyclo(3.3.0)octan, 3-(4-Chlorphenoxy)-1,2-propandiol, Hyamine, Alkyl-($C_8$-$C_{18}$)-dimethyl-benzyl-ammoniumchlorid, Alkyl-($C_8$-$C_{18}$)-dimethyl-benzylammonium-bromid, Alkyl-($C_8$-$C_{18}$)-dimethyl-benzylammoniumsaccharinat, Benzylhemiformal, 3-Jod-2-propinyl-butylcarbamat, Natrium-hydroxymethyl-aminoacetat oder Natrium-hydroxymethyl-aminoacetat.

Sollen die erfindungsgemäßen Mischungen vornehmlich zur Inhibition des Wachstums unerwünschter Mikroorganismen auf oder in tierischen Organismen eingesetzt werden, so ist eine Kombination mit weiteren antibakteriellen oder antimykotischen Wirkstoffen auch hier in manchen Fällen vorteilhaft. Erwähnenswert sind insoweit als weitere Wirkstoffe neben der großen Gruppe der klassischen Antibiotika insbesondere die für Kosmetika relevanten Produkte wie Triclosan, Climbazol, Octoxyglycerin, Octopirox (1-Hydroxy-4-methyl-6-(2,4,4-trimethylpentyl)-2(1H)-pyridone, 2-aminoethanol), Chitosan, Farnesol, Glycerinmonolaurat, aliphatische und aromatische Hydroxamsäuren, Tropolon, Hinokitiol oder Kombinationen der genannten Substanzen, die u.a. gegen Achselgeruch, Fußgeruch oder Schuppenbildung eingesetzt werden.

Die erfindungsgemäßen Mischungen können, insbesondere in kosmetischen Zubereitungen, vorteilhaft mit weiteren üblichen Bestandteilen kombiniert werden, wie beispielsweise:
Weitere Konservierungsmittel, weitere antimikrobielle Mittel wie z.B weitere anitbakterielle Mittel oder Fungizide, Abrasiva, Antiakne-Mittel, Mittel gegen Hautalterung, Anticellulitis-Mittel, Antischuppen-Mittel, entzündungshemmende Mittel, irritationsverhindernde Mittel, irritationshemmende Mittel, Antioxidantien, Adstringentien, schweisshemmende Mittel, antiseptische Mittel, Antistatika, Binder, Puffer, Trägermaterialien, Chelatbildner, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Slilcone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, $\alpha$-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Darüber hinaus können die erfindungsgemäßen Mischungen auch in Kombination mit schweißhemmenden Wirkstoffen (Antitranspirantien) besonders vorteilhaft zur Bekämpfung von Körpergeruch eingesetzt werden. Als schweißhemmende Wirkstoffe kommen vor allem Aluminiumsalze wie Aluminiumchlorid, Aluminiumchlorhydrat, -nitrat, -sulfat, -acetat usw. zum Einsatz. Daneben kann aber auch die Verwendung von Zink-, Magnesium- und Zirkoniumverbindungen vorteilhaft sein. Für die Anwendung in kosmetischen und dermatologischen Antitranspirantien haben sich im Wesentlichen die Aluminiumsalze und - in etwas geringerem Maße - Aluminium/Zirkoniumsalz-Kombinationen bewährt. Daneben erwähnenswert sind die teilneutralisierten und damit besser hautverträglichen, aber nicht ganz so wirksamen Aluminiumhydroxychloride.

Sollen die erfindungsgemäßen Mischungen zur antimikrobiellen Behandlung einer Oberfläche (z.B. eines menschlichen oder tierischen Körpers) eingesetzt werden, so ist in manchen Fällen eine Kombination mit (Metall)-Chelatoren vorteilhaft. Bevorzugt einzusetzende (Metall)-Chelatoren sind hierbei u.a $\alpha$-Hydroxyfettsäuren, Phytinsäure, Lactoferrin, $\alpha$-Hydroxysäuren wie u.a. Zitronensäure, Milchsäure und Äpfelsäure sowie Huminsäuren, Gallensäuren, Gallenextrakte, Bilirubin, Biliverdin bzw. EDTA, EGTA und deren Derivate.

Zur Anwendung werden die erfindungsgemäßen kosmetischen und/oder dermatologisch wirksamen Mischungen in der für Kosmetika und Dermatika üblichen Weise auf die Haut und/oder die Haare in ausreichender Menge aufgebracht. Besondere Vorteile bieten dabei kosmetische und dermatologische Zubereitungen, die eine erfindungsgemäße Mischung enthalten und zusätzlich als Sonnenschutzmittel wirken. Vorteilhaft enthalten diese Zubereitungen mindestens einen UVA-Filter und/oder mindestens einen UVB-Filter und/oder mindestens ein anorganisches Pigment.

Die Zubereitungen können dabei in verschiedenen Formen vorliegen, wie sie z.B. üblicherweise für Sonnenschutzzubereitungen eingesetzt werden. So können sie z.B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, einen festen Stift oder auch ein Aerosol darstellen.

Wie erwähnt können Zubereitungen, die eine erfindungsgemäße Mischung enthalten, vorteilhaft mit Substanzen kombiniert werden, die UV-Strahlung absorbieren, wobei die Gesamtmenge der Filtersubstanzen z.B. 0,01 Gew.-% bis 40 Gew.-%, vorzugsweise 0,1% bis 10 Gew.-%, insbesondere 1,0 bis 5,0 Gew.-% beträgt, bezogen auf das Gesamtgewicht der Zubereitungen, um kosmetische Zubereitungen zur Verfügung zu stellen, die das Haar bzw. die Haut vor ultravioletter Strahlung schützen.

In erfindungsgemäße Mischungen enthaltenden Formulierungen zur topischen prophylaktischen oder kosmetischen Behandlung der Haut ist regelmäßig ein hoher Anteil an pflegenden Substanzen vorteilhaft. Gemäß einer bevorzugten Ausführungsform enthalten die Zusammensetzungen ein oder mehrere pflegende tierische und/oder pflanzliche Fette und Öle wie Olivenöl, Sonnenblumenöl, raffiniertes Sojaöl, Palmöl, Sesamöl, Rapsöl, Mandelöl, Borretschöl, Nachtkerzenöl, Kokosöl, Sheabutter, Jojobaöl, Spermöl, Rindertalg, Klauenöl und Schweineschmalz sowie gegebenenfalls weitere pflegende Bestandteile wie zum Beispiel Fettalkohole mit 8-30 C-Atomen.

Zu pflegenden Substanzen, die sich vorzüglich mit den erfindungsgemäßen synergistischen Mischungen kombinieren lassen, zählen darüber hinaus auch

- Ceramide, wobei man unter Ceramiden N-Acylsphingosine (Fettsäreamide des Sphingosins) oder synthetische Analoga solcher Lipide (sogenannte Pseudo-Ceramide) versteht, die das Wasserhaltevermögen des Stratum Corneums deutlich verbessern.

- Phospholipide, beispielsweise Sojalecithin, Ei-Lecithin und Kephaline

- Vaseline, Paraffin- und Silikonöle; zu letzteren zählen unter anderem Dialkyl- und Alkylarylsiloxane wie Dimethylpolysiloxan und Methylphenylpolysiloxan, sowie deren alkoxylierte und quaternierte Derivate.

Kosmetische Zubereitungen, die erfindungsgemäße Mischungen enthalten, können auch Antioxidantien enthalten, wobei alle für kosmetische und/oder dermatologische Anwendungen geeigneten oder gebräuchlichen Antioxidantien verwendet werden können.

Kosmetische Zubereitungen, die erfindungsgemäße Mischungen enthalten, können auch Vitamine und Vitaminvorstufen enthalten, wobei alle für kosmetische und/oder dermatologische Anwendungen geeigneten oder gebräuchlichen Vitamine und Vitaminvorstufen verwendet werden können. Erwähnenswert sind hier insbesondere Vitamine und Vitaminvorstufen wie Tocopherole, Vitamin A, Niacinsäure und Niacinsäureamid, weitere Vitamine des B-Komplexes, insbesondere Biotin und Vitamin C, Panthenol und dessen Derivate, insbesondere die Ester und Ether des Panthenols sowie kationisch derivatisierte Panthenole wie z.B. Panthenoltriacetat, Panthenolmonoethylether und dessen Monoacetat sowie kationische Panthenolderivate.

Kosmetische Zubereitungen, die erfindungsgemäße Mischungen enthalten, können auch entzündungshemmende bzw. rötungs- bzw. juckreizlindernde Wirkstoffe enthalten. Es können hierbei alle für kosmetische und/oder dermatologische Anwendungen geeigneten oder gebräuchlichen entzündungs-hemmenden bzw. rötungs- und juckreizlindernden Wirkstoffe verwendet werden.

Kosmetische Zubereitungen, die erfindungsgemäße Mischungen enthalten, können auch Wirkstoffe mit hautaufhellender oder hautbräunender Wirkung enthalten. Erfindungsgemäß können hierbei alle für kosmetische und/oder dermatologische Anwendungen geeigneten oder gebräuchlichen hautaufhellenden oder hautbräunenden Wirkstoffe verwendet werden.

Kosmetische Zubereitungen, die erfindungsgemäße Mischungen enthalten, können auch anionische, kationische, nichtionische und/oder amphotere Tenside enthalten, zumal wenn kristalline oder mikrokristalline Festkörper, beispielsweise anorganische Mikropigmente in die Zubereitungen eingearbeitet werden sollen.

Die Erfindung wird nachfolgend anhand von Beispielen näher erläutert. Sofern nicht anders angegeben beziehen sich die Angaben auf das Gewicht.

**BEISPIELE**

**Beispiel 1 (Referenzbeispiel)**

**Synergistische Wirksamkeit der erfindungsgemäßen ternären Mischungen**

Es wurde ein Vergleich auf ausreichende Konservierung kosmetischer Formulierungen enthaltend 2-Phenoxyethanol (Produkt A, nicht erfindungsgemäß), 1,2-Decandiol (Produkt B, nicht erfindungsgemäß), 1,2-Hexandiol (Produkt C, nicht erfindungsgemäß), eine binäre Mischung aus 2-Phenoxyethanol und 1,2-Hexandiol (Produkt D, nicht erfindungsgemäß), eine binäre Mischung aus 2-Phenoxyethanol und 1,2-Decandiol (Produkt E, nicht erfindungsgemäß), eine binäre Mischung aus 1,2-Decandiol und 1,2-Hexandiol (Produkt F, nicht erfindungsgemäß) und eine ternäre Mischung aus 2-Phenoxyethanol, 1,2-Decandiol und 1,2-Hexandiol (Produkt G, erfindungsgemäß) durchgeführt.

Die Prüfung auf ausreichende Konservierung erfolgte gemäß Europäischem Arzneibuch.

Die Prüfung besteht somit aus der Kontamination der Zubereitung, wenn möglich in ihrem Endverhältnis, mit einem vorgeschriebenen Inokulum geeigneter Mikroorganismen, der Lagerung der beimpften Zubereitung bei einer bestimmten Temperatur, der Entnahme der Proben aus dem Behältnis in bestimmten Zeitabständen und der Bestimmung der Anzahl der Mikroorganismen in den so entnommenen Proben. Die konservierenden Eigenschaften sind ausreichend, wenn sich unter den Bedingungen der Prüfung eine eindeutige Verminderung oder gegebenenfalls keine Vermehrung der Keimzahl in den beimpften Zubereitungen nach den vorgeschriebenen Zeiten bei den vorgeschriebenen Temperaturen ergibt. Experimentelle Details der Versuchsdurchführung sind im Europäischen Arzneibuch (ISBN 3-7692-2768-9; Nachtrag 2001 zur 3. Ausgabe, Seite 421-422, Kapitel 5.1.3) beschrieben.

Testkeime:

Folgende Mikroorganismen-Stämme wurden für die Tests auf ausreichende Konservierung verwendet:

A: *Escherichia coli* ATCC 8739

B: *Pseudomonas aeruginosa* ATCC 9027

C: *Staphylococcus aureus* ATCC 6538

D: *Candida albicans* ATCC 10231

E: *Aspergillus niger* ATCC 16404

Die anfängliche Keimzahl (**KBE**/g; **K**olonie **B**ildende **E**inheiten/g; "0"-Wert") lag bei den unterschiedlichen Testreihen im Bereich von 1.000.000 bis 1.200.000.

Formulierung:

Für die Tests auf ausreichende Konservierung wurde die erfindungsgemäße Wirkstoffkombination (Produkt G) in definierter Menge in eine O/W-Emulsion eingearbeitet. Zu Vergleichszwecken wurden die Vergleichsprodukte (Produkt A bis F) in separate O/W-Emulsionen eingearbeitet.

**Tabelle 1**

Formulierungen mit Produkten A bis G (Mengenangaben als Gew.-%)

| Phase A | INCI-Name | Hersteller | mit | | | | | | |
|---|---|---|---|---|---|---|---|---|---|
| | | | "A" | "B" | "C" | "D" | "E" | "F" | "G" |
| Dracorin CE 614035 | Glyceryl Stearate Citrate | Symrise | 4,0 | 4,0 | 4,0 | 4,0 | 4,0 | 4,0 | 4,0 |
| Dracorin GMS 647834 | Glyceryl Stearate | Symrise | 1,5 | 1,5 | 1,5 | 1,5 | 1,5 | 1,5 | 1,5 |
| Lanette E | Stearyl Alcohol | BASF | 1,5 | 1,5 | 1,5 | 1,5 | 1,5 | 1,5 | 1,5 |
| PCL Solid 660086 | Stearyl Heptanoate, Stearyl Caprylate | Symrise | 3,0 | 3,0 | 3,0 | 3,0 | 3,0 | 3,0 | 3,0 |
| Paraffinöl °E | Paraffinum Liquidum | Parafluid | 6,0 | 6,0 | 6,0 | 6,0 | 6,0 | 6,0 | 6,0 |
| Dow Corning 345 | Cyclopentasiloxane, Cyclohexasiloxane | Dow Corning | 2,0 | 2,0 | 2,0 | 2,0 | 2,0 | 2,0 | 2,0 |
| **Phase B** | | | | | | | | | |
| Wasser, entmineralisiert | Water (Aqua) | | Ad 100 | | | | | | |
| Carbopol ETD 2050 Polymer | Carbomer | Noveon | 0,15 | 0,15 | 0,15 | 0,15 | 0,15 | 0,15 | 0,15 |
| 1,2 Hexandiol | 1,2 Hexanediol | Symrise | - | - | 1,0 | 0,2 | - | 0,45 | 0,1 |
| 1,2-Decandiol | Decylene Glycol | Symrise | - | 1,0 | - | - | 0,25 | 0,55 | 0,2 |
| 2-Phenoxyethanol | Phenoxyethanol | Symrise | 1,0 | - | - | 0,8 | 0,75 | - | 0,7 |
| **Phase C** | | | | | | | | | |
| Neutralizer AMP-95 | Amino Methylpropanol | Dow/Angus | 0,1 | 0,1 | 0,1 | 0,1 | 0,1 | 0,1 | 0,1 |
| **Summe:** | | | 100,0 | 100,0 | 100,0 | 100,0 | 100,0 | 100,0 | 100,0 |
| pH-Wert: 5,5 | | | | | | | | | |

Ergebnis:

Die Ergebnisse der Konservierungsmittel-Belastungstests für *Aspergillus niger* zu den untersuchten Wirkstoffkombinationen bestehend aus der erfindungsgemäßen Mischung (Produkt G) bzw. den Vergleichssystemen (Produkte A und F) sind in Tabelle 2 gegenübergestellt. Hier zeigt sich der synergistische Effekt der erfindungsgemäßen Mischung (Produkt G) in den bereits nach 48 Stunden verbleibenden Rest-Keimzahlen für *Aspergillus niger*. Wie aus der Tabelle ersichtlich ist, konnte bei *Aspergillus niger,* einem hinsichtlich der Konservierung industrieller Produkte besonders problematischen Keim, die Keimzahl durch Verwendung der erfindungsgemäßen Mischung in einer Dosierung von 1 Gew.-% innerhalb von 48 Stunden von 1.200.000 auf 14.000 reduziert werden. Die zu Vergleichszwecken in einer Dosierung von 1 Gew.-% getesteten Vergleichsprodukte A bis F ermöglichten bei *Aspergillus niger* hingegen keine derartig signifikante Reduktion der Anzahl koloniebildender Einheiten (KBE). Diese Testreihe zeigt somit beispielhaft, dass erfindungsgemäße ternäre Wirkstoffgemische eine synergistisch gegenüber den Produkten A bis G verbesserte Wirkung besitzen.

Hinsichtlich der weiteren Testkeime wurden ebenfalls hervorragende Ergebnisse erhalten, welche die Überlegenheit des erfindungsgemäßen Produktes G bestätigen.

Berechnung der synergistisch antimikrobiellen Wirkung einer ternären Mischungen enthaltend 2-Phenoxyethanol, 1,2-Decandiol und 1,2-Hexandiol

Zur Bestimmung der potentiell synergistisch verstärkten Wirksamkeit einer ternären Mischungen enthaltend 2-Phenoxyethanol, 1,2-Decandiol und 1,2-Hexandiol, wurden für die Einzelstoffe, für verschiedene binäre Mischungen sowie für erfindungsgemäße ternäre Mischungen die KBE-reduzierenden Eigenschaften gemäß der in Beispiel 1 beschriebenen Versuchsdurchführung ermittelt. Die Ergebnisse der Untersuchungen sind in Tabelle 2 gelistet.

**Tabelle 2**

| Prüfung auf *Aspergillus niger* KBE-Reduktion für 2-Phenoxyethanol (Produkt A, nicht erfindungsgemäß), 1,2-Decandiol (Produkt B, nicht erfindungsgemäß), 1,2-Hexandiol (Produkt C, nicht erfindungsgemäß), eine binäre Mischung aus 2-Phenoxyethanol und 1,2-Hexandiol (Produkt D, nicht erfindungsgemäß), eine binäre Mischung aus 2-Phenoxyethanol und 1,2-Decandiol (Produkt E, nicht erfindungsgemäß), eine binäre Mischung aus 2-Phenoxyethanol und 1,2-Hexandiol (Produkt F, nicht erfindungsgemäß) und eine ternäre Mischung aus 2-Phenoxyethanol, 1,2-Decandiol and 1,2-Hexandiol (Produkt G, erfindungsgemäß) sowie errechnete Synergie-Index Werte für ausgewählte binäre und ternäre Mischungen | | | | |
|---|---|---|---|---|
| | **Gesamt-Einsatzkonzentration [Gew. %]** | **Aspergillus niger initiale KBE** | **Aspergillus niger KBE nach 48h** | **Synergie-Index SI** |
| 1 Gew.-% 2-Phenoxyethanol (Produkt A) | 1 | 1200000 | 1100000 | ./. |
| 1 Gew.-% 1,2-Decandiol (Produkt B) | 1 | 1200000 | 1200000 | ./. |
| 1 Gew.-% 1,2-Hexandiol (Produkt C) | 1 | 1200000 | 1000000 | ./. |
| 0,70 Gew.-% 2-Phenoxyethanol und 0,20 Gew.-% 1,2-Decandiol und 0,10 Gew.-% 1,2-Hexandiol (Produkt (G) | 1 | 1200000 | 14000 | 0,0129 |

Ergebnis:

Die Berechnung des Synergieindex (SI-Werte) der erfindungsgemäßen ternären Mischungen sowie nicht erfindungsgemäßer binärer Mischungen nach Kull (Literatur: F.C.Kull et al, Applied Microbiology Vol.9, p. 538-541 (1961); D.C.Steinberg, Cosmetics & Toiletries Vol.115(11), 59-62 (2000) erfolgte mittels folgender Gleichungen.

Binäre Mischungen: $SI = Z * D/A + Z * E/B$

Ternäre Mischung: SI = Z * D/A + Z * E/B + Z * F/C

wobei:

D,E,F : Faktor Mengenanteil der Einzelbestandteile (Bsp: 70 Gewichtsprozent = Faktor 0.70)

A, B, C: KBE der Einzelstoffe zum Zeitpunkt 48h

Z: KBE der binären/ternären Mischung zum Zeitpunkt 48h

Ein synergistischer Effekt liegt vor, wenn Werte kleiner 1.00 erhalten werden. Werte größer 1.00 zeigen einen antagonistischen Effekt. Bei einem Wert von exakt 1.00 liegt weder ein synergistischer noch ein antagonistischer Effekt vor.

Nachfolgend ist die Berechnung des Synergie-Index für die erfindungsgemäße ternäre Mischung enthaltend 0,70 Gew.-% 2-Phenoxyethanol, 0,20% 1,2-Decandiol und 0,10 Gew.-% 1,2-Hexandiol aufgeführt.

$$SI = 14.000 * 0.70/1.100.000 + 14.000 * 0.20/1.200.000 + 14.000 * 0.10/1.000.000 = 0,0126$$

Der SI-Wert von **0,0126** zeigt eine hochsignifikante, synergistisch verstärkte Wirkung der Mischung enthaltend 2-Phenoxyethanol, 1,2-Decandiol und 1,2-Hexandiol an.

Die in Tabelle 2 gelisteten Ergebnisse und hier insbesondere der errechneten Synergie Indices zeigen eindeutig, dass die erfindungsgemäße ternäre Mischung (Produkt G) enthaltend 2-Phenoxyethanol, 1,2-Decandiol und 1,2-Hexandiol eine deutlich synergistisch verstärkte antimikrobielle Wirkung aufweist. Im Fall der binären Mischungen (Produkte D - F) konnte kein signifikanter Synergie-Effekt nachgewiesen werden, im Gegenteil, in einigen Fällen war sogar ein negativer Synergieeffekt (ein verstärktes Keimwachstum) verglichen mit dem Einsatz der Mischungen A, B und C zu beobachten.

Hieraus lässt sich schließen, dass der überraschend deutliche Synergieeffekt in der ternären Mischung wiederum überraschenderweise nicht auf einem Effekt einer Mischung von lediglich zwei Komponenten des 3-Komponentensystems beruht.

Ein synergistischer Effekt wie in Beispiel 1 dargestellt konnte in einem weiteren Beispiel, für eine ternäre Mischungen enthaltend 2-Phenoxyethanol, 1,2-Decandiol und 1,2-Pentandiol ersatzweise für 1,2-Hexandiol erzielt werden. Die synergistische Wirkungsverstärkung dieser ternären Mischungen kann auf eine bessere Verfügbarkeit von Phenoxyethanol und 1,2-Decandiol in der Wasserphase durch den Zusatz von 1,2-Hexandiol oder 1,2-Pentandiol zurückzuführen sein. Kurzkettige antimikrobiell wirksame Diole wie 1,2-Hexandiol oder 1,2-Pentandiol können als Lösungsvermittler dienen, die die Konzentration der deutlich stärker antimikrobiell wirksamen Substanzen 2-Phenoxyethanol und 1,2-Decandiol in der Wasserphase von Emulsionen, dem Lebensraum der Mikroorganismen, erhöhen.

### Beispiel 2

### Kosmetische/dermatologische Anwendungsbeispiele

Zur Anwendung werden die synergistisch wirksame ternäre Mischungen enthaltenden dermatologischen und kosmetischen Zubereitungen in Kombination mit anderen kosmetischen Wirk- und Zusatzstoffen in der für Kosmetika üblichen Weise auf die Haut und/oder die Haare in ausreichender Menge aufgebracht. Nachfolgend sind beispielhaft vorteilhafte Zubereitungen für einige Anwendungen angegeben:

**Hautfeuchte regulierende Creme (nicht erfindungsgemäß)**

| Phase | Inhaltsstoff | INCI | Gew.-% | Gew.-% |
|---|---|---|---|---|
| A | Dracorin CE | Glyceryl stearate citrate | 4.00 | |
| A | Cutina GMS | Glyceryl stearate | 1.50 | 1.50 |
| A | Lanette 18 | Stearyl alcohol | 1.50 | 3.00 |
| A | PCL Solid | Stearyl heptanoate, stearyl caprylate | 3.00 | 2.00 |

(fortgesetzt)

| Phase | Inhaltsstoff | INCI | Gew.-% | Gew.-% |
|---|---|---|---|---|
| A | Emulsiphos | Potassium Cetyl Phosphate, Hydrogenated Palm Glycerides | | 1.50 |
| A | Mineral Oil | Mineral oil | 6.00 | 4.00 |
| A | Dow corning 345 | Cyclopentasiloxane, Cyclohexasiloxane | 2.00 | 2.00 |
| A | Dragoxat 89 | Ethylhexyl Isononanoate | | 4.00 |
| B | Water | Water | 77.15 | 78.60 |
| B | Carbopol ETD 2050 | Carbomer | 0.25 | |
| B | Disodium EDTA | Disodium EDTA | 0.10 | 0.10 |
| B | Xanthan gum | | | 0.30 |
| C | NaOH 10% Lösung | NaOH 10% solution | 0.50 | |
| C | Zemea | 1,3-Propanediol | 2.00 | |
| C | Hydrolite 5 | Pentylene glycol | | 2.00 |
| C | SymGlucan | Water, Glycerin, Beta-Glucan | 1.00 | |
| D | 2-Phenoxyethanol, 1,2-Decandiol, 1,2-Hexandiol (7:2:1)* | Phenoxyethanol, Decylene Glycol, 1,2-Hexanediol | 1.00 | 0.60 |
| D | Fragrance | Fragrance | | 0.40 |

* Referenzbeispiel

**Sonnenschutz Formulierung (nicht erfindungsgemäß)**

| Phase | Inhaltsstoff | INCI | Gew.-% | Gew.-% |
|---|---|---|---|---|
| A | Neo Heliopan BB | Benzophenone-3 | 6.00 | 1.50 |
| A | Lanette 18 | Stearyl alcohol | 1.50 | 1.00 |
| A | Neo Heliopan HMS | Homosalate | 10.00 | |
| A | Neo Heliopan OS | Ethylhexyl Salicylate | 5.00 | |
| A | Neo Heliopan 357 | Avobenzone | 3.00 | 1.50 |
| A | Neo Heliopan AV | Ethylhexyl Methoxycinnamate | 7.50 | |
| A | Neo Heliopan 303 | Octocrylene | | 10.00 |
| A | Tocopheryl Acetate | Tocopheryl Acetate | | 0.20 |
| A | Ethylhexyl Triazone | Ethylhexyl Triazone | | 1.00 |
| A | PCL Solid | Stearyl heptanoate, stearyl caprylate | 2.00 | |
| A | Emulsiphos | Potassium Cetyl Phosphate, Hydrogenated Palm Glycerides | 2.00 | |
| A | Tegosoft TN | C12-15 Alkyl Benzoate | 3.00 | |
| A | Neutral Oil | Caprylic/Capric triglyceride | 3.00 | |
| A | Dragoxat 89 | Ethylhexyl Isononanoate | 2.00 | |
| A | Xanthan gum | | 0.20 | |
| A | Tegosoft XC | Phenoxyethyl Caprylate | | 5.00 |

(fortgesetzt)

| Phase | Inhaltsstoff | INCI | Gew.-% | Gew.-% |
|---|---|---|---|---|
| A | Tegosoft DC | Decyl Cocoate | | 2.50 |
| B | Butylene glycol | Butylene glycol | | 2.00 |
| B | Tego Care CG 90 | Cetearyl Glucoside | | 1.00 |
| B | Water | Water | 51.10 | 72.35 |
| B | Carbopol ETD 2050 | Carbomer | 0.20 | 0.05 |
| B | Disodium EDTA | Disodium EDTA | 0.10 | 0.10 |
| B | Tego Carbomer 341 ER | Acrylates / C10-30 Alkyl Acrylate Crosspolymer | | 0.05 |
| C | AMP Ultra | Aminomethyl Propanol | 0.15 | 0.10 |
| C | Glycerin | Glycerin | 2.00 | |
| D | 2-Phenoxyethanol, 1,2-Decandiol, 1,2-Hexandiol (7:2:1)* | Phenoxyethanol, Decylene Glycol, 1,2-Hexanediol, | 1.00 | 1.25 |
| D | Parfümöl | Fragrance | 0.25 | 0.40 |
| * Referenzbeispiel | | | | |

**Shampoo Formulierung (nicht erfindungsgemäß)**

| Phase | Inhaltsstoff | INCI | Gew.-% | Gew.-% |
|---|---|---|---|---|
| A | Water | Water | 50.70 | 57.90 |
| A | Aculy 88 | Acrylates/Steareth-20 Methacrylate Crosspolymer | | 3.45 |
| A | Novethix L-10 | Acrylates/Beheneth-25 Methacrylate Copolymer | 2.50 | |
| B | Plantapon SF | Sodium Cocoamphoacetate, Glycerin, Laureyl Glucoside, Sodium Cocoyl Glutamate, Sodium Lauryl Glucose Carboxylate | 30.00 | |
| B | Plantacare 2000 UP | Decyl Glucoside | 3.00 | |
| B | Lamesoft PO 65 | Coco-Glucoside, Glyceryl Oleate | 5.00 | |
| B | Stepanol AMV | Ammonium Lauryl Sulfate | | 10.00 |
| B | Sodium Laureth Sulphate 28% | Sodium Laureth Sulphate | | 10.00 |
| C | Dehyton AB 30 | Coco Betaine | 7.00 | |
| C | Triethanolamine | Triethanolamine | | 1.50 |
| C | Mackanate EL 40% | Disodium Laureth Sulfosuccinate | | 8.00 |
| C | Cocamidopropyl Betaine 30% | Cocamidopropyl Betaine | | 4.00 |
| C | PEG-12 Dimethicone | PEG-12 Dimethicone | | 2.00 |
| C | Disodium EDTA | Disodium EDTA | 0.10 | 0.10 |
| C | Polyquaternium-10 | Polyquaternium-10 | | 0.25 |
| C | NaOH 10% solution | NaOH 10% solution | 0.20 | |
| C | Propylene Glycol | Propylene Glycol | | 2.00 |

(fortgesetzt)

| Phase | Inhaltsstoff | INCI | Gew.-% | Gew.-% |
|---|---|---|---|---|
| D | 2-Phenoxyethanol, 1,2-Decandiol, 1,2-Hexandiol (7:2:1)* | Phenoxyethanol, Decylene Glycol, 1,2-Hexanediol | 1.00 | 0.80 |
| D | Parfümöl | Fragrance | 0.50 | |
| * Referenzbeispiel | | | | |

**Formulierungen für die dekorative Kosmetik (nicht erfindungsgemäß)**

| Phase | Inhaltsstoff | INCI | Gew.-% | Gew.-% |
|---|---|---|---|---|
| A | Abil Wax 2440 | Behenoxy Dimethicone | 2.00 | |
| A | Isodragol | Triisononanoin | | 2.00 |
| A | Cetearyl Ethylhexanoate | Cetearyl Ethylhexanoate | 5.50 | |
| A | Rewopal PIB 1000 | Polyisobutene | 5.00 | |
| A | Dragoxat 89 | Ethylhexyl Isononanoate | 8.00 | |
| A | Talc | Talc | | 4.00 |
| A | PVP/Eicosene Copolymer | PVP/Eicosene Copolymer | 0.25 | |
| A | Polydecene | Polydecene | | 3.00 |
| A | Titanium Dioxide | Titanium Dioxide | | 6.00 |
| A | Iron Oxides | Iron Oxides | | 1.60 |
| B | Myristyl Lactate | Myristyl Lactate | 8.00 | |
| B | Candelilla Wax | Candelilla Wax | 7.50 | |
| B | Copernicia Cerifera (Carnauba) Wax | Copernicia Cerifera (Carnauba) Wax | 2.50 | |
| B | Dimethyl Isosorbide | Dimethyl Isosorbide | 3.00 | |
| B | Covapate Unired LC3728 | Ricinus Communis (Castor) Seed Oil, CI 45410 | 3.90 | |
| B | Ceresin | Ceresin | 3.00 | |
| B | Covapate Uniwhite LC7981 | Ricinus Communis (Castor) Seed Oil, CI 77891 | 9.60 | |
| B | Rubis Covapate W4765 | Ricinus Communis (Castor) Seed Oil, CI 15850; | 1.45 | |
| B | Brun Covapate W8760 | Ricinus Communis (Castor) Seed Oil, Iron Oxides | 5.50 | |
| B | Lanolin Oil | Lanolin Oil | 18.00 | |
| B | Lanolin | Lanolin | 16.00 | |
| B | Tocopheryl Acetate | Tocopheryl Acetate | 0.20 | |
| B | Cyclopentasiloxane, Cyclohexasiloxane | Cyclopentasiloxane, Cyclohexasiloxane | | 18.00 |
| B | Abil EM 97 | Bis-PEG/PPG-14/14 Dimethicone | | 2.80 |
| B | Phenyl Trimethicone | Phenyl Trimethicone | | 1.00 |
| C | Water | Water | | 53.55 |

14

(fortgesetzt)

| Phase | Inhaltsstoff | INCI | Gew.-% | Gew.-% |
|---|---|---|---|---|
| C | Sodium Chloride | Sodium Chloride | | 1.25 |
| C | Propylene Glycol | | | 6.00 |
| C | 2-Phenoxyethanol, 1,2-Decandiol, 1,2-Hexandiol (7:2:1)* | Phenoxyethanol, Decylene Glycol, 1,2-Hexanediol | 0.50 | 0.80 |
| C | Parfümöl | Fragrance | 0.10 | |
| * Referenzbeispiel | | | | |

**Beispiel 3**

**Weitere Formulierungen enthaltend erfindungsgemäße ternäre antimikrobielle Mischungen**

In der nachfolgenden Tabelle bedeuten

3.1 = Hautaufhellende Tagescreme O/W

3.2 = Hautberuhigende Lotion mit Pflanzenextrakten O/W

3.3 = After Sun Balm

3.4 = Körperspray

3.5 = Sonnenschutzlotion (O/W), Breitbandschutz

3.6 = W/O Nachtcreme

3.7 = Shampoo

3.8 = Selbstbräunungscreme

3.9 = Barrierereparaturcreme O/W

3.10 = Antitranspirant/Deodorant Roll-on

Mengenangaben in Gew.-%

| Rohmaterial (Hersteller) | INCI | 3.1 | 3.2 | 3.3 | 3.4 | 3.5 | 3.6 | 3.7 | 3.8 | 3.9 | 3.10 |
|---|---|---|---|---|---|---|---|---|---|---|---|
| 2-Phenoxyethanol | Phenoxyethanol | 0.7 | 0.7 | 1.0 | 0.7 | 1.4 | 0.7 | 0.7 | 1.05 | 0.7 | 0.7 |
| SymClariol (Symrise) | Decylene Glycol | 0.2 | 0.2 | 0.4 | 0.2 | 0.4 | 0.2 | 0.2 | 0.3 | 0.2 | 0.2 |
| Hydrolite-6 (Symrise) | 1,2-Hexanediol | 0.1 | | 0.2 | 0.1 | | | 0.1 | | 0.1 | |
| Hydrolite-5 (Symrise) | Pentylene Glycol | | 0.1 | | | 0.2 | 0.1 | 0.1 | 0.15 | | 0.1 |
| -(Alpha-)-Bisabolol, natural (Symrise) | Bisabolol | 0.3 | 0.4 | 0.3 | 0.1 | 0.3 | 0.2 | 0.05 | 0.2 | 0.5 | 0.1 |
| Ingwer CO$_2$ Extrakt (Flavex) | Zingiber Officinale (Ginger) Root Extract | 0.003 | 0.004 | 0.003 | 0.005 | 0.003 | 0.002 | 0.001 | 0.002 | 0.01 | 0.001 |
| Abil 350 (Degussa-Goldschmidt) | Dimethicone | 0.5 | 2.0 | 1.0 | | | | | 0.5 | 0.5 | |
| Allantoin (Merck) | Allantoin | | 0.2 | 0.1 | | | | | | | |
| Aloe Vera Gel Concentrate 10/1 (Symrise) | Water (Aqua), Aloe Barbadensis Leaf Juice | | | 3.0 | | | 3.0 | | | | |
| Alugel 34 TH (Baerlocher) | Aluminium Stearate | | | | | | 1.0 | | | | |
| Aqua-Ceramide (Kao) | Cetyloxypropyl Glyceryl Methoxypropyl Myristamide | | 0.1 | | | | | | | | 0.1 |
| Arbutin (Sabinsa) | β-Arbutin | 1.0 | | | | | | | | | |
| Natrium Ascorbyl Phosphate (EMD Chemicals) | Natrium Ascorbyl Phosphate | 2.0 | | 1.0 | | | | | | | |
| Butylene Glycol | Butylene Glycol | | | 5.0 | | | | | | | |
| Carbopol ETD 2050 (Noveon) | Carbomer | | | | | 0.2 | | | | | |
| Carbopol Ultrez-10 (Noveon) | Carbomer | | 0.1 | | | | | | | | |
| Ceramide 2 (Sederma) | Ceramide 2 | 0.1 | | | | | | | | | |
| Ceramide PC104 (Pacific Corporation) | Hydroxypropyl Bispalmitamide MEA | | | | | 0.1 | | | | | |

| | | 3.1 | 3.2 | 3.3 | 3.4 | 3.5 | 3.6 | 3.7 | 3.8 | 3.9 | 3.10 |
|---|---|---|---|---|---|---|---|---|---|---|---|
| Ceramide SL (Sino Lion) | Hydroxyethyl Palmityl Oxyhydroxypropyl Palmitamide | | | | | | 0.1 | | | | |
| Cetiol OE (Cognis) | Dicaprylyl Ether | | | 4.0 | | | | | | | |
| Cetiol SB 45 (Cognis) | Butyrospermum Parkii (Shea Butter) | | | 1.0 | | | | | | | |
| Citric Acid 10% sol. | Citric Acid | | | | | | 0.3 | | | | |
| Comperlan 100 (Cognis) | Cocamide MEA | | | | | | 0.5 | | | | |
| Dihydroxyaceton (Merck) | Dihydroxyacetone | | | | | | | | 5.0 | | |
| Dow Corning 246 Fluid (Dow Corning) | Cyclohexasiloxane and Cyclopentasiloxane | | | | | 2.0 | | | | | |
| Dow Corning 345 Fluid (Dow Corning) | Cyclomethicone | | | | 0.5 | | | | | | |
| D-Panthenol (BASF) | Panthenol | | | 1.0 | | | | | | | |
| Dracorin CE (Symrise) | Glyceryl Stearate Citrate | 5.0 | | | | | | | 5.0 | 1.5 | |
| Dracorin GMS (Symrise) | Glyceryl Stearate | | 2.0 | | | | | | | 2.0 | |
| Dracorin GOC (Symrise) | Glyceryl Oleate Citrate, Caprylic/Capric Triglyceride | | | | 2.0 | | | | | | |
| Drago-Beta-Glucan (Symrise) | Water (Aqua), Butylene Glycol, Glycerin, Avena Sativa (Oat), Kernel Extract | 0.3 | | | | | | | | | |
| Dragocid Liquid (Symrise) | Phenoxyethanol, Methylparaben, Ethylparaben, Butylparaben, Propylparaben, Isobutylparaben | | 0.8 | 0.7 | | 0.7 | 0.8 | | | 0.8 | |
| Dragoderm (Symrise) | Glycerin, Triticum Vulgare (Wheat) Gluten, Water (Aqua) | | | | | | | 2.0 | | | |
| Drago-Oat-Active (Symrise) | Water (Aqua), Butylene Gylcol, Avena Sativa (Oat) Kernel Extract | | | | 1.0 | | | | | | |
| Dragosan W/O Liquid (Symrise) | Polyglyceryl-3-Polyricinoleate, Sorbitan Isostearate | | | | | | 1.0 | | | | |

EP 2 589 291 B2

(fortgesetzt)

| | 3.1 | 3.2 | 3.3 | 3.4 | 3.5 | 3.6 | 3.7 | 3.8 | 3.9 | 3.10 |
|---|---|---|---|---|---|---|---|---|---|---|
| Dragosan W/O P (Symrise) — Sorbitan Isostearate, Hydrogenated Castor Oil, Ceresin, Beeswax (Cera Alba) | | | | | | 6.0 | | | | |
| Dragoxat EH (Symrise) — Ethylhexy Ethylhexanoate | 3.0 | 3.0 | | 4.0 | | | | 3.0 | | |
| Dragoxat 89 (Symrise) — Ethylhexyl Ethylisononanoate | | | | | | | | | 2.0 | |
| EDETA B Pulver (BASF) — Tetrasodium EDTA | | | | | | | 0.1 | | | |
| EDETA DB (BASF) — Disodium EDTA | | | | | 0.1 | | | 0,1 | | |
| Emulsiphos (Symrise) — Potassium Cetyl Phosphate, Hydrogenated Palm Glycerides | | 2.0 | | | 1.5 | | | | 2.0 | |
| Ethanol 96 % — Ethanol | | | | | | | | 2.0 | | 30.0 |
| Extrapone Green Tea GW (Symrise) — Glycerin, Water (Aqua), Camellia Sinensis Leaf Extract | | 0.2 | | | | | | | | |
| Extrapone Witch Hazel Distillate colorless (Symrise) — Propylene Glycol, Hamamelis Virginiana (Witch Hazel) Water (Aqua), Hamamelis Virginiana (Witch Hazel) Extract | | | | | | 1.0 | | | | |
| Extrapone Rosemary GW (Symrise) — Glycerin, Water (Aqua), Rosmarinus officinalis (Rosemary) Leaf Extract | | 0.3 | | | | | | | 0.5 | |
| Farnesol (Symrise) — Farnesol | | | | | | | | | | 0.5 |
| Frescolat ML crist. (Symrise) — Menthyl Lactate | | | 0.8 | | | | | | | |
| Genapol LRO liquid (Cognis) — Sodium Laureth Sulfate | | | | | | | 37.0 | | | |
| Givobio GZN (Seppic) — Zinc Gluconate | | | | | | | | | 0.5 | |
| Glycerin 85 % — Glycerin | 3.0 | 2.0 | 4.0 | | 4.7 | 2.0 | | 1.5 | 3.0 | |
| Hydroviton (Symrise) — Water, Glycerin, Sodium Lactate, TEA Lactate, Serine, Lactic Acid, Urea, Sorbitol, Sodium Chloride, Lauryl Diethylenediaminoglycine, Lauryl Aminopropylglycine, Allantoin | | | | | | | | | 1.0 | |
| Irgasan DP 300 (Ciba Geigy) — Triclosan | | | | | | | | | | 0.3 |

(fortgesetzt)

| | | 3.1 | 3.2 | 3.3 | 3.4 | 3.5 | 3.6 | 3.7 | 3.8 | 3.9 | 3.10 |
|---|---|---|---|---|---|---|---|---|---|---|---|
| Isodragol (Symrise) | Triisononanoin | | 2.0 | | | | | | | 3.0 | |
| Isopropylpalmitat (Symrise) | Isopropyl Palmitate | 4.0 | | | | | | | 4.0 | | |
| Karion F (Merck) | Sorbitol | | | | | | 2.0 | | | | |
| Keltrol RD (CP-Kelco) | Xanthan Gum | 0.2 | 0.1 | | | | | | | | |
| Keltrol T (Danby-Chemie) | Xanthan Gum | | | | | 0.2 | | | 0.3 | | |
| Kojic acid (Cosmetochem) | Kojoc acid | 1.0 | | | | | | | | | |
| Lanette 16 (Cognis) | Cetyl Alcohol | 1.0 | | | | | | | 1.0 | | |
| Lanette O (Cognis) | Cetearyl Alcohol | | 3.0 | | | 1.0 | | | | 2.0 | |
| Lara Care A-200 (Rahn) | Galactoarabinan | | | 0.3 | | | | | | | |
| Magnesium Chloride (Merck) | Magnesium Chloride | | | | | | 0.7 | | | | |
| Merquat 550 (Ondeo Nalco) | Polyquaternium-7 | | | | | | | 0.5 | | | |
| NAOH 10% sol. | Sodium Hydroxide | | | | | | | | | 0.3 | |
| Naringin (Exquim) | 4',5,7-Trihydroxyflavon-7-O-neohesperidosid | | | | | | | 0.5 | 2.0 | | |
| Natriumbenzoat | Sodium Benzoate | | | | | | | 0.5 | | | |
| Natrosol 250 HHR (Aqualon) | Hydroxyethylcellulose | | | | | | | | | | 0.3 |
| Neo Heliopan 357 (Symrise) | Butyl Methoxy-dibenzoylmethane | | | | | 1.0 | | | | | |
| Neo Heliopan AP (Symrise) (10 % als Natriumsalz) | Disodium Phenyl Dibenzimidazole Tetrasulfonate | | | | | 10 | | | | | |
| Neo Heliopan AV (Symrise) | Ethylhexyl Methoxycinnamate | | | | | 3.0 | | | | | |
| Neo Heliopan Hydro (Symrise) (15 % als Natriumsalz) | Phenylbenzimidazole Sulfonic Acid | | | | | 6.7 | | | | | |
| Neo Heliopan MBC (Symrise) | 4-Methylbenzylidene Camphor | | | | | 1.5 | | | | | |

(fortgesetzt)

| | | 3.1 | 3.2 | 3.3 | 3.4 | 3.5 | 3.6 | 3.7 | 3.8 | 3.9 | 3.10 |
|---|---|---|---|---|---|---|---|---|---|---|---|
| Neo Heliopan OS (Symrise) | Ethylhexyl Salicylate | | | | | 5.0 | | | | | |
| Neutral Oil | Caprylic/Capric Triglyceride | 6.0 | | | 4.0 | 2.0 | | | 6.0 | 10.0 | |
| Oxynex 2004 (Merck) | BHT | | | | | | 0.1 | | | | |
| Paraffinöl 5 Grad E (Parafluid) | Paraffinum Liquidum | | | | 4.0 | | | | | | |
| PCL Liquid 100 (Symrise) | Cetearyl Ethylhexoate | 3.0 | 5.0 | | 7.0 | | | | | | |
| PCL Solid (Symrise) | Stearyl Heptanoate, Stearyl Caprylate | | 2.0 | | | | | | | | |
| PCL-Liquid (Symrise) | Cetearyl Ethylhexanoate, Isopropyl Myristate | | | | | | 12.0 | | 3.0 | | |
| Pemulen TR-2 (Noveon) | Acrylates/C10-30 Alkyl Acrylate Crosspolymer | | | 0.3 | 0.2 | | | | | | |
| 4-(1-Phenylethyl)1,3-benzenediol | 4-(1-Phenylethyl)1,3-benzenediol | 0.5 | | | | | | | | | |
| Propylene Glycol-1,2 99P GC | Propylene Glycol | | 5.0 | | | | | | | | |
| Pseudoceramide 391 | N-(1-Hexadecanoyl)-4-hydroxy-L-prolin-(1-hexadecyl -ester | | 0.1 | | | | | 0.2 | | 0.5 | |
| Retinyl Palmitate in Oil (DSM Nutrional Products) | Retinyl Palmitate | | | | | | 0.2 | | | | |
| Sepigel 305 | Polyacrylamide, C13-14 Isoparaffin, Laureth-7 | | | | | | | | 1.0 | | |
| Sodium Chloride | Sodium Chloride | | | | | | | 1.0 | | | |
| Sodium Hydroxide (10% sol. | Sodium Hydroxide | | 0.3 | 0.6 | 0.4 | | | | | | |
| Solubilizer 611674 (Symrise) | PEG-40 Hydrogenated Castor Oil, Trideceth-9, Water (Aqua) | | | | | | | | | | 2.0 |
| Sun Flower Oil (Wagner) | Helianthus Annuus (Sunflower) Seed Oil | | | | | | 5.0 | | | | |
| Sweet Almond Oil (Wagner) | Prunus dulcis | | | | | | 5.0 | | | | |
| Symdiol 68 (Symrise) | 1,2 Hexanediol, Caprylylglycol | 0.5 | | | | | | | | | |

EP 2 589 291 B2

(fortgesetzt)

|  |  | 3.1 | 3.2 | 3.3 | 3.4 | 3.5 | 3.6 | 3.7 | 3.8 | 3.9 | 3.10 |
|---|---|---|---|---|---|---|---|---|---|---|---|
| Symrise Fragrance | Fragrance | 0.3 | 0.3 | 0.3 | 0.2 | 0.4 | 0.4 | 0.5 | 0.3 | 0.3 | 1.0 |
| Tamasterol (Tama Biochemicals) | Phytosterols | | | | | | | | | 0.3 | |
| Tego Betain L7 (Degussa) | Cocamidopropyl Betain | | | | | | | 6.0 | | | |
| Tegosoft PC 31(Degussa) | | | | | | | | | | 0.3 | |
| Tegosoft TN (Degussa) | C12-15 Alkyl Benzoate | | | 5.0 | | 5.0 | | | | | |
| Triethanolamine, 99% | Triethanolamine | | | | | 0.5 | | | | | |
| Tocopherol Acetate (DSM Nutritional Products) | Tocopheryl Acetate | | | 0.5 | | 0.5 | 3.0 | | | 0.3 | |
| Zirkonal L 450 (BK Giulini) | Aluminium Zirconium Pentachlorohydrate (40%ige wässrige Lösung) | | | | | | | | | | 37.0 |
| Water, demineralized | Wasser (Aqua) | ad 100 | ad 100 | ad 100 | ad 100 | ad 100 | ad 100 | ad 100 | ad 100 | ad 100 | ad 100 |

**Patentansprüche**

1. Verwendung einer Mischung, umfassend oder bestehend aus:

   (a) 2-Phenoxyethanol,
   (b1) 1,2-Decandiol und
   (b2) 1,2-Pentandiol,

   zur Konservierung und antimikrobiellen Behandlung verderblicher Produkte gegen *Aspergillus niger,* wobei das Gewichtsverhältnis der Komponente (a) zur Komponente (b1 + b2) 0,3 - 1,2 zu 0,06 - 0,7 beträgt.

2. Verwendung nach Anspruch 1, wobei die Gewichtsverhältnisse der Komponenten (a) zu (b1) zu (b2) 0,3 -1,2 zu 0,05 - 0,4 zu 0,01 - 0,3 betragen.

3. Kosmetische oder pharmazeutische Zubereitung oder Nahrungsmittel enthaltend eine Mischung bestehend aus

   (a) 0,30 bis 1,20 Gew.- % 2-Phenoxyethanol
   (b1) 0,05 bis 0,40 Gew.- % 1,2-Decandiol und
   (b2) 0,01 bis 0,20 Gew.- % 1,2- Pentandiol,

   bezogen auf die Gesamtmasse der Zubereitung oder des Nahrungsmittels.

4. Kosmetische oder pharmazeutische Zubereitung oder Nahrungsmittel nach Anspruch 3 wobei die Konzentration jeder der Komponenten (a), (b1) und (b2) für sich unterhalb der antimikrobiell wirksamen Konzentration liegt, aber die Gesamtkonzentration der Komponenten (a), (b1) und (b2) antimikrobiell wirksam ist.

5. Verwendung von 2-Phenoxyethanol zur synergistischen Verstärkung der antimikrobiellen Wirksamkeit einer Mischung aus Komponenten (b1) und (b2) gegen *Aspergillus niger,* wobei das Gewichtsverhältnis der Komponente (a) zur Komponente (b1 + b2) 0,3 - 1,2 zu 0,06 - 0,7 beträgt.

6. Verfahren zur Konservierung oder antimikrobiellen Behandlung eines verderblichen Produktes gegen *Aspergillus niger,* mit folgendem Schritt: Kontaktieren des verderblichen Produktes mit einer antimikrobiell wirksamen Menge einer Mischung nach einem der Ansprüche 1 und/oder 2.

7. Mischung umfassend oder bestehend aus:

   (a) 2-Phenoxyethanol,
   (b1) 1,2-Decandiol und
   (b2) 1,2-Pentandiol,

   wobei das Gewichtsverhältnis der Komponente (a) zur Komponente (b1 + b2) 0,3 - 1,2 zu 0,06 - 0,7 beträgt, zur therapeutischen Behandlung von

   (i) Körpergeruch verursachenden Mikroorganismen,
   (ii) Akne verursachenden Mikroorganismen und/oder
   (iii) Mykosen verursachenden Mikroorganismen,

   wobei die Mikroorganismen *Aspergillus niger* sind.

**Claims**

1. Use of a mixture comprising or consisting of:

   (a) 2-phenoxyethanol,
   (b1) 1,2-decanediol and
   (b2) 1,2-pentanediol,

for the preservation and antimicrobial treatment of perishable products against *Aspergillus niger,* wherein the weight ratio of component (a) to component (b1 + b2) is 0.3 - 1.2 to 0.06-0.7.

**2.** Use according to claim 1, wherein the weight ratios of components (a) to (b1) to (b2) are 0.3 -1.2 to 0.05 - 0.4 to 0.01 - 0.3.

**3.** Cosmetic or pharmaceutical preparation or food containing a mixture consisting of

(a) 0.30 to 1.20 % by weight of 2-phenoxyethanol
(b1) 0.05 to 0.40% by weight of 1,2-decanediol and
(b2) 0.01 to 0.20% by weight of 1,2- pentanediol,

calculated on the total mass of the preparation or food.

**4.** Cosmetic or pharmaceutical preparation or food according to claim 3 wherein the concentration of each of components (a), (b1) and (b2) is below the antimicrobially effective concentration but the total concentration of components (a), (b1) and (b2) is antimicrobially effective.

**5.** Use of 2-phenoxyethanol for synergistic enhancement of the antimicrobial activity of a mixture of components (b1) and (b2) against *Aspergillus niger,* wherein the weight ratio of component (a) to component (b1 + b2) is 0.3 - 1.2 to 0.06 - 0.7.

**6.** A method for preserving or antimicrobially treating a perishable product against Aspergillus niger, comprising the step of: contacting the perishable product with an antimicrobially effective amount of a mixture according to one of Claims 1 and/or 2.

**7.** Mixture comprising or consisting of:

(a) 2-phenoxyethanol,
(b1) 1,2-decanediol and
(b2) 1,2-pentanediol,

wherein the weight ratio of component (a) to component (b1 + b2) is 0.3 - 1.2 to 0.06 - 0.7, for the therapeutic treatment of

(i) micro-organisms causing body odour,
(ii) acne-causing micro-organisms and/or
(iii) micro-organisms causing mycoses,

wherein the microorganisms are Aspergillus niger.


**Revendications**

**1.** Utilisation d'un mélange comprenant ou consistant en :

(a) 2-phénoxyéthanol,
(b1) 1,2-décanediol et
(b2) 1,2-pentanediol,

pour la conservation et le traitement antimicrobien de produits périssables contre *Aspergillus niger,* le rapport pondéral du composant (a) au composant (b1 + b2) étant de 0,3 - 1,2 à 0,06 - 0,7.

**2.** Utilisation selon la revendication 1, dans laquelle les rapports de poids des composants (a) à (b1) à (b2) sont de 0,3 -1,2 à 0,05 - 0,4 à 0,01 - 0,3.

**3.** Préparation cosmétique ou pharmaceutique ou aliment contenant un mélange consistant en

(a) 0,30 à 1,20 % en poids de 2-phénoxyéthanol
(b1) 0,05 à 0,40 % en poids de 1,2-décanediol et de
(b2) 0,01 à 0,20 % en poids de 1,2-pentanediol,

par rapport à la masse totale de la préparation ou de l'aliment.

4. Préparation cosmétique ou pharmaceutique ou un aliment selon la revendication 3, dans laquelle la concentration de chacun des composants (a), (b1) et (b2) est inférieure à la concentration efficace du point de vue antimicrobien mais la concentration totale des composants (a), (b1) et (b2) est efficace sur le plan antimicrobien.

5. Utilisation de 2-phénoxyéthanol pour l'amélioration synergique de l'activité antimicrobienne d'un mélange de composants (b1) et (b2) contre Aspergillus ni-ger, dans laquelle le rapport pondéral du composant (a) au composant (b1 + b2) est 0,3 - 1,2 à 0,06 - 0,7.

6. Procédé de conservation ou de traitement antimicrobien d'un produit périssable contre Aspergillus niger, comprenant l'étape consistant à : mettre en contact le produit périssable avec une quantité efficace du point de vue antimicrobien d'un mélange selon une des revendications 1 et/ou 2.

7. Mélange comprenant ou consistant en :

(a) 2-phénoxyéthanol,
(b1) 1,2-décanediol et
(b2) 1,2-pentanediol,

dans laquelle le rapport pondéral du composant (a) au composant (b1 + b2) est de 0,3 - 1,2 à 0,06 - 0,7, pour le traitement thérapeutique de

(i) micro-organismes responsables des odeurs corporelles,
(ii) des micro-organismes responsables de l'acné et/ou
(iii) les micro-organismes responsables des mycoses,

dans laquelle les micro-organismes sont Aspergillus niger.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 5191327 B
- JP 11322591 B
- EP 1206933 A
- WO 03069994 A
- WO 2005102276 A1
- WO 03069994 A1
- DE 20221386 U1

**In der Beschreibung aufgeführte Nicht-Patentliteratur**

- Deutsche Gesellschaft für wissenschaftliche und angewandte Kosmetik. Handbuch der Konservierungsmittel. Verlag für chemische Industrie, 1995
- *Ausgabe,* 2001, vol. 5.1.3, 421-422
- **F.C.KULL et al.** *Applied Microbiology,* 1961, vol. 9, 538-541
- **D.C.STEINBERG.** *Cosmetics & Toiletries,* 2000, vol. 115 (11), 59-62